# FASCICULE DE BREVET EUROPEEN

(11) **EP 2 874 672 B1**
(45) Date de publication et mention de la délivrance du brevet: **07.09.2016**
(21) Numéro de dépôt: 13744758.7
(22) Date de dépôt: 16.07.2013
(51) Int. Cl.: A61L 27/20, A61L 27/52

(54) **HYDROGEL DE CHITOSANE POUR LA REPARATION DU TISSU NERVEUX**
CHITOSAN-HYDROGEL ZUR REPARATUR VON NERVENGEWEBE
CHITOSAN HYDROGEL FOR REPAIRING NERVE TISSUE

(30) Priorité: 19.07.2012 FR 1257006
(43) Date de publication de la demande: 27.05.2015
(73) Titulaire: Université Claude Bernard Lyon I, 69622 Villeurbanne Cedex (FR); Institut National Des Sciences Appliquées De Lyon, 69621 Villeurbanne Cedex (FR); Université Jean Monnet de Saint-Etienne, 42023 Saint-Etienne Cedex 2 (FR); Centre National de la Recherche Scientifique (CNRS), 75794 Paris Cedex 16 (FR); INSERM - Institut National de la Santé et de la Recherche Médicale, 75654 Paris Cedex (FR); Université Pierre et Marie Curie (Paris 6), 75252 Paris Cedex 05 (FR)
(72) Inventeur: NOTHIAS, Fatiha, F-75005 Paris (FR); SOARES, Sylvia, F-93260 Les Lilas (FR); DAVID, Laurent, F-69004 Lyon (FR); MONTEMBAULT, Alexandra, F-42000 Saint Etienne (FR)
(74) Mandataire: Sarlin, Laure V.
(86) Numéro de dépôt international: PCT/FR2013/051710
(87) Numéro de publication internationale: WO 2014/013188

(56) Documents cités:
- CHO ET AL.: "Chitosan nanoparticle-based neuronal membrane sealing and neuroprotection following acrolein-induced cell injury", JOURNAL OF BIOLOGICAL ENGINEERING, vol. 4, no. 2, 2010, pages 1-11, XP002695806,
- YING YUAN ET AL.: "The interaction of Schwann cells with chitosan membranes and fibers in vitro", BIOMATERIALS, vol. 25, 2004, pages 4273-4278, XP002695803,
- KARIN S. STALEY ET AL.: "Biomaterial design strategies for the treatment of spinal cord injuries", JOURNAL OF NEUROTRAUMA, vol. 27, 2010, pages 1-19, XP002695805,

## Description

La présente invention concerne le domaine général de la régénération axonale à travers une cicatrice gliale, en particulier produite par un traumatisme du système nerveux central (SNC) ou périphérique (SNP) tel que les lésions traumatiques de la moelle épinière.

Les lésions traumatiques de la moelle épinière (ME) conduisent à une paralysie, une perte de la sensation et une douleur chronique. Dans la grande majorité des cas chez l'homme (comme chez le rongeur dans les modèles expérimentaux), ces lésions se traduisent par la rupture de connexions nerveuses entre le cerveau et le reste du corps, suivie par une neurodégénérescence progressive à partir du site lésionnel et la formation d'une cavité entourée d'une cicatrice gliale. L'ensemble constitue une barrière à la fois physique et chimique pour la repousse des axones. La barrière chimique est due à une importante production par les cellules non neuronales de molécules inhibitrices (cytokines, protéoglycans (i.e. CSPG (Chondroitin sulfate proteoglycans) de la matrice extracellulaire et de molécules chémorépulsives). D'autres molécules inhibitrices proviennent de la myéline (myelin-derived protein Nogo-A, MAG (Myelin-Associated Glygoprotein et Omgp Oligodendrocyte myelin glycoprotein); elles sont exposées dans la MEC suite à la démyélinisation des axones. L'apport en facteurs trophiques reste malheureusement faible. Ainsi, la réparation du réseau neuronal rompu est bloquée.

En plus de ces effets inhibiteurs, la biodisponibilité de facteurs trophiques favorisant la survie et la régénération axonale est assez limitée. En comparaison, le système nerveux périphérique est plutôt susceptible d'une réparation et d'une régénération axonale, après une lésion traumatique, grâce à la réponse rapide inflammatoire des macrophages et l'action des cellules de Schwann (cellules myélinisantes du SNP), en faveur d'une élimination rapide des débris cellulaires et de la myéline. Cependant, dans le cas des lésions sévères, la régénération complète est quelquefois limitée en raison de la difficulté à guider les fibres vers les cibles périphériques appropriées.

Néanmoins, le SNC des mammifères est doté d'une grande plasticité neuronale. Ainsi, après une lésion partielle de la ME, les axones épargnés peuvent subir des remaniements et participer à une récupération fonctionnelle, liée essentiellement à une pousse de prolongements collatéraux (ou "sprouting"). Ce sont ces observations qui ont déclenché depuis, le développement de stratégies thérapeutiques pour la réparation du système nerveux endommagé (Nothias F (2011) Stimuler la repousse axonale, in "Régénération nerveuse, des cellules souches aux interfaces cerveau-machine"; spécial issue magazine, BIOFUTUR VOL 30/322 JUIN2011, pp.36-40).

Plusieurs modèles expérimentaux ont été développés pour stimuler la repousse axonale en luttant contre l'action inhibitrice des protéines contenues dans la cicatrice gliale:
- Injection de l'anticorps IN1 pour antagoniser l'effet inhibiteur de certaines composantes de la myéline (Nogo, MAG et Omgp) qui partagent le même récepteur au niveau du neurone, Nogo-A.
- Dégradation par une enzyme spécifique (ChABC, une Chondroitinase de type ABC) au niveau de la matrice extracellulaire du protéoglycan CSPG, inhibiteur également de la pousse axonale.
- Injection de peptide mimant l'effet de molécules d'adhérence favorable pour la pousse axonale. C'est le cas de peptide mimant la fonction de l'oligosaccharide PSA (acide polysialique) qui dans le cerveau est lié à la molécule d'adhérence NCAM (neural cell adhésion molecule). La NCAM au cours du développement est riche en PSA et joue un rôle primordial pour la croissance et la fasciculation des faisceaux de fibres.
- Manipulation générique pour réduire la réaction gliale particulièrement celle des astrocytes,
- Apport de facteurs neurotrophiques (BDNF (Brain-derived neurotrophic factor), NT3 (Neurtrophin 3)) ou de cellules ayant un effet trophique sur les neurones pour augmenter le taux de survie et également pour promouvoir une repousse axonale.
- Substitution du tissus lésé par une transplantation de neurones embryonnaires, de cellules souches, cellules de Schwann (cellules myélinisantes du système nerveux périphérique) ou encore des cellules engainantes du bulbe olfactif pour guider et éventuellement myéliniser les axones,

Dans la majorité des modèles expérimentaux de réparation de la moelle épinière (ME) traumatique, seul un nombre faible d'axones régénère et peu d'entre eux arrivent à traverser l'ensemble du site lésionnel Deux obstacles majeurs subsistent qu'il faudrait lever :
(i) il est nécessaire de contrôler la fenêtre temporelle pour une repousse axonale efficace : il faut agir le plus rapidement possible après la lésion ;
(ii) il faut prendre en compte l'organisation spatiale du site lésionnel pour offrir non seulement un milieu permissif mais également une organisation spatiale contribuant au guidage de la repousse.

Des matériaux polymères bioactifs et biodégradables, naturels ou synthétiques, ont été également utilisés pour créer un support physique et mécanique à la croissance atonale, et/ou mimer l'effet biologique d'une matrice extracellulaire. C'est le collagène, un composant majeur de la matrice extracellulaire, sous la forme d'hydrogel, qui a été le plus utilisé. Cependant, alors que son implantation dans le SNP a abouti à une meilleure régénération axonale, au niveau du SNC seule sa combinaison avec un apport de facteurs trophiques a montré une amélioration fonctionnelle partielle. En outre, les implants polymères utilisés devaient en général avoir une forme permettant de guider la régénération atonale (ex : implants tubulaires sous forme de bridges).

Le chitosane est un polymère bioactif et biodégradable connu dans l'état de la technique, notamment pour ses propriétés de biocompatibilité, de bio-résorbabilité et cicatrisantes. Ces propriétés ont été utilisées, en médecine et en chirurgie, entre autres.

Le chitosane est un polymère d'origine naturelle obtenu par désacétylation de la chitine. La chitine est un biopolymère de haut poids moléculaire, non-toxique et biodégradable, et c'est, avec la cellulose, le polysaccharide le plus répandu dans la nature. La chitine est constituée d'une chaîne linéaire dont la structure contient des unités de répétition de type glucosamine (GLcN) et N-acétylglucosamine (GLcNAc) liées par une liaison β,(1->4). L'extraction de la chitine à partir de carapaces de crustacés et/ou d'endosquelettes de calamars, se fait principalement par voie chimique, puis la transformation par désacétylation de la chitine dans de la soude concentrée à chaud permet d'obtenir le chitosane. De par sa biocompatibilité avec les tissus humains et ses propriétés cicatrisantes, le chitosane a démontré son efficacité sous diverses formes physiques telles que des gels, poudres ou films, dans divers produits tels que des pansements, emplâtres, peau artificielle, pansement cornéen, fils de suture en chirurgie qui se résorbent naturellement après cicatrisation, mais aussi dans la réparation osseuse ou dans la réparation de cartilage (Montembault et al Biochimie, 88, 2006, pages 551-564) et en chirurgie dentaire, dans les implants ou dans la cicatrisation des gencives, dans des lentilles de contact bien tolérées.

Le chitosane a également été utilisé comme implant intrathécal sous vertébral dans les colonnes vertébrales de rats (Kim Howard et al. Journal of Biomedical Materials Research, 15 juin 2011, vol. 97A, numéro 4, pages 395-404). Toutefois, l'implant utilisé avait la forme d'une feuille non poreuse et le test mis en oeuvre était destiné à montrer que ce biopolymère était biocompatible et pouvait donc être utilisé pour des applications à long terme dans la réparation de la moelle épinière. Ce document ne décrit donc pas de repousse axonale dans le cas de l'utilisation du chitosane sous forme de feuilles non poreuses. En outre, il est fort probable qu'une telle repousse soit impossible, vu la forme du chitosane utilisé.

Dans l'article de synthèse de Karin S. Straley et al, (J Neurotrauma. 2010 January; 27(1): 1-19 dol: 10.1089/neu.2009.0948), il est indiqué que des échafaudages de chitosane ont été utilisés pour transférer des greffes de nerfs viables, des cellules souches neurales, et des cellules progénitrices neurales dans la colonne vertébrale de rats, ce qui a favorisé la régénération axonale. Toutefois, dans ces applications le chitosane n'est jamais utilisé seul et est toujours sous forme de blocs. En outre le chitosane est cité parmi de nombreux autres biomatériaux tels que le collagène, les Poly α-hydroxy acides, l'acide hyaluronique et la fibrine, sans que le chitosane ne soit indiqué comme particulièrement plus intéressant que ces autres matériaux.

D'une façon générale, les hydrogels utilisés dans le développement de stratégies thérapeutiques pour la moelle épinière ou nerf périphérique, ont été conçus essentiellement sous forme de tubes à large diamètre, comme support pour y adhérer et confiner des cellules favorables pour la repousse axonale comme les cellules de Schwann. Par ailleurs, plusieurs de ces études ont utilisé le chitosane mélangé avec des protéines ou des polysaccharides tels que la gélatine, collagène, poly-D-lysine ou encore de l'agarose ; le chitosane ayant la plus faible concentration dans la mixture (Zuidema et al., 2011, Acta Biomateriala 7:1634-1643*;* Karin et al., 2010, journal of Neurotrauma, 27:1-19 *;* Annabi et al., Tissue Eng Part B Rev. 2010, 16:371-383*; revue de* Yang T-L, 2011, Int. J. Mol.Sci, 12, 1936-1963*;* Pfister LA et al., 2007, J Biomed Mater Res A. 80:932-7).

La publication de Youngnam Cho et al. dans Biological engineering 2010, 4, 2 intitulée « Chitosan nanoparticle-based neuronal membrane sealing and neuroprotection following acrolein-induced cell injury» décrit l'utilisation de nanoparticules particules de complexes formés de chitosane. et de sulfate de dextran ou de tripolyphosphate de sodium, dans lesquels est incorporé ou non de l'hydralazine et évalue leur utilisation dans un modèle *in vitro* d'effet neuro-protecteur. Compte-tenu de la taille des particules décrites dans ce document, qui est de 250-300nm, il est démontré que les particules sont internalisées dans les cellules cultivées *in vitro* (comme illustré sur la **Figure 9**).

De façon surprenante, les inventeurs ont découvert que des microparticules d'hydrogel de chitosane pouvaient être utilisées avec succès comme implant dans la régénération axonale, à condition que le chitosane ait un faible degré d'acétylation, sans avoir besoin de conférer à l'implant une forme permettant de guider les axones, tels que les tubes. En effet, l'implant à base de microparticules a une forme physique ouverte et poreuse, plus importante que ce que peut offrir un bloc. Les résultats obtenus chez le rat montrent que ces microparticules d'hydrogel peuvent être utilisées avec succès en particulier dans la réparation des lésions de la moelle épinière puisque les axones traversent le site lésionnel sur une longue distance (3 à 4 mm au moins). Les inventeurs ont découvert que l'introduction du chitosane réduit la réaction des astrocytes, à la fois morphologique et moléculaire. En effet, ces cellules sont connues pour réagir rapidement à l'introduction d'un élément qui ne fait pas partie du tissu nerveux, elles forment une barrière avec leurs prolongements de façon à créer une frontière entre le tissu endommagé et le tissu sain. Elles sécrètent aussi des molécules dont la plupart sont inhibitrices pour la repousse axonale (exemple le CSPG), Par ailleurs, les inventeurs ont découvert que même les astrocytes peuvent migrer à travers l'implant et leur prolongement à la frontière de la lésion est associé aux axones qui repoussent, un phénomène qui est décrit au cours du développement du système nerveux. Un tel effet sur les astrocytes et les axones n'avait jamais été démontré dans les modèles expérimentaux utilisés auparavant: le nombre d'axones obtenus dans ces modèles était insuffisant pour envahir l'ensemble de la lésions, autour des 10%. Les inventeurs ont découvert que le chitosane n'a pas uniquement des propriétés adhésives mais également attractives. Ils ont noté que la rétraction des axones est très réduite, ce qui explique les nombreuses fibres qui repoussent à travers la lésion,

La présente invention concerne donc des microparticules d'hydrogel de chitosane de taille médiane d50 pour une distribution en nombre comprise entre 1 et 500 µm, le chitosane ayant un degré d'acétylation inférieur ou égal à 20% et sa concentration dans l'hydrogel étant comprise entre 0,25 et 5% en poids par rapport au poids total de l'hydrogel, pour une utilisation dans la régénération neuronale et/ou dans la réparation du système nerveux (central ou périphérique), avantageusement du système nerveux central (en particulier la moelle épinière), et/ou dans la greffe de neurones et/ou dans le traitement des maladies neurodégénératives (telles que la maladie d'Alzheimer ou de Parkinson) et/ou dans le traitement des paralysies.

Avantageusement, la réparation ou la régénération ou le traitement des paralysies fait suite à une lésion, en particulier traumatique du système nerveux (central ou périphérique telles que les lésions sévères du nerf périphérique), encore plus avantageusement du système nerveux central, en particulier du cerveau ou, de préférence, de la moelle épinière,
En effet, les microparticules sont implantées, soit au niveau de la lésion, en particulier de la lésion traumatique, afin de combler cette lésion, soit à l'endroit où la repousse axonale ou la réparation ou la greffe doit avoir lieu. Les microparticules sont donc avantageusement implantées par injection, de façon encore plus avantageuse par chirurgie.

Le traitement peut ne pas faire suite à une lésion traumatique comme dans le cas des lésions neurodégénératives. Ces lésions neurodégénératives ne sont pas dues à un impact physique. Elles entraînent la perte de neurones dans des sites précis. Les axones en provenance d'autres régions se retrouvent alors sans cellules cibles et ne peuvent donc plus jouer leur rôle. D'une part, l'implantation au niveau de la lésion des microparticules selon la présente invention peut créer un milieu favorable pour stimuler leur repousse vers d'autres neurones non endommagés. D'autre part, les neurones connectés au départ à la région lésée voyant un déficit d'entrée d'information afférente, l'implantation de particules peut également stimuler la repousse de collatérales des axones voisins.

Le chitosane utilisé dans le cadre de l'invention est obtenu par désacétylation de la chitine en milieu alcalin (Robert GAF Chitin Chemistry, Macmillan Press Ltd Ed., London 1992), la chitine étant le polymère de structure des exosquelettes des arthropodes (A. Domard et G. Chaussard; New Approach in the Study of The Production of Chitosan from Squid Pens: Kinetics, Thermodynamic and Structural Aspects. In Adv. Chitin Sc. 5, 1-5. 2002*;* K. Kurita et al.; Squid Chitin as a Potential Alternative Chitin Source: Deacetylation Behavior and Characteristic Properties. J. Polym. Sci. Part A 31, 485-491. 1993), des endosquelettes des céphalopodes (H.-M. Cauchie; An Attempt to Estimate Crustacean Chitin Production in the Hydrosphere. In Adv. Chitin Sci. 2, 32-39, 1992) ou encore des parois cellulaires de certains champignons ou algues. Par conséquent, hormis dans les cas où le degré d'acétylation est de 0%, le chitosane est un copolymère des deux monomères suivantes : le 2-acétamido-2-désoxy-D-glucopyranose et le 2-amino-2-désoxy-D-glucopyranose liés par une liaison glycosidique β,(1->4). En particulier, le chitosane est disponible dans le commerce, par exemple chez Mahtani Chitosan par désacétylation de la chitine de calamar ou de crevette.

Le chitosane utilisable dans le cadre de la présente invention possède un degré d'acétylation inférieur ou égal à 20%, c'est-à-dire compris entre 0 et 20%. En effet, si un degré d'acétylation plus important est utilisé, les inventeurs se sont aperçus que le chitosane ne permet pas la régénération axonale et en outre, induit une réaction inflammatoire importante. Avantageusement, le chitosane utilisable dans le cadre de la présente invention possède un degré d'acétylation compris entre 0 et 10%, de façon avantageuse inférieur à 5%, de façon encore plus avantageuse compris entre 1 et 4%, en particulier d'environ 3%. Le degré d'acétylation influe, en outre, sur la biorésorbabilité du chitosane, lorsque celui-ci est implanté dans un organisme, c'est à dire sa dégradation dans l'organisme, en particulier le corps humain, qui aboutit à sa disparition, ses produits de dégradation étant métabolisés ou excrétés par l'organisme. En effet, plus le degré d'acétylation croît, plus la cinétique de biodégradation et de bio-résorption est élevée. Celui-ci sera donc choisi en fonction de l'application visée. Dans le cadre de la présente invention degré d'acétylation du chitosane est mesuré en utilisant la technique de RMN du proton, en suivant la méthodologie d'Hirai (Asako Hirai, Hisashi Odani, Akio Nakajima, Polymer Bulletin (1991) Volume: 26, Issue: 1, Publisher: Springer, Pages: 87-94).

Dans un mode de réalisation particulier la masse molaire du chitosane est supérieure à 180 000 g/mol, avantageusement supérieure à 200 000 g/mol, de façon avantageuse supérieure à 300 000 g/mol, de façon plus avantageuse inférieure à 1 000 000 g/mol, de façon encore plus avantageuse inférieure à 600 000 g/mol, en particulier d'environ 450 000 g/mol, Les inventeurs se sont aperçus que, de façon surprenante, les hautes masses molaires permettent d'obtenir un hydrogel peu concentré en chitosane tout en limitant la réponse inflammatoire, et en favorisant la biorésorbabilité de l'hydrogel

Par hydrogel, on entend au sens de la présente invention une masse viscoélastique (ayant un module de cisaillement complexe G*=G"+jG" tel que G'>10G" sur une gamme de fréquence supérieure à 1 décade) comportant au moins 80% en masse d'eau, de préférence au moins 90% en masse d'eau, et préférentiellement au moins 95% en masse d'eau. Il existe deux catégories d'hydrogels : les hydrogels chimiques et les hydrogels physiques. Un hydrogel est dit physique, lorsque les interactions responsables de la réticulation Inter-chaînes sont de type physique, et sont notamment des liaisons hydrogène et/ou des interactions hydrophobes par opposition à un hydrogel dit chimique dans lequel les interactions inter-chaines sont de type liaison covalente.

Dans le cadre de la présente invention la concentration du chitosane dans l'hydrogel est comprise entre 0,25 et 5% en poids par rapport au poids total de l'hydrogel, avantageusement inférieure à 4 % en poids par rapport au poids total de l'hydrogel, de façon avantageuse supérieure à 0,5% en poids par rapport au poids total de l'hydrogel, en particulier compris entre 1 et 3% en poids par rapport au poids total de l'hydrogel, plus particulièrement d'environ 2,5% en poids par rapport au poids total de l'hydrogel, Les inventeurs se sont aperçus que lorsque la solution de chitosane possède une concentration inférieure à 0,25% en poids, il n'est pas possible de gélifier la solution de façon à obtenir un hydrogel macroscopique. En outre, au-delà de 5% en poids de chitosane dans la solution, sa dissolution devient difficile et la viscosité des solutions obtenue rend difficile l'élaboration des matériaux.

Dans un mode de réalisation avantageux de la présente invention, on utilisera un hydrogel physique de chitosane, L'avantage de ce type d'hydrogel est qu'il ne contient pas de réticulant chimique qui peut être toxique. La formation de tels hydrogels est, par exemple décrite dans les publications suivantes, auxquelles on pourra se référer : A. Montembault; C. Viton et A. Dornard dans Rheometric study of the gelation of chitosan in a hydroalcoholic medium. Biomateria/s, 26(14), 1633-1643, 2004 *et* Montembault A, Viton C, Dornard A, Rheometric study of the gelation of chitosan in aqueous water without cross-linking agent, Biomacromolecules, 6(2): 653-662, 2005*.*

En particulier, l'hydrogel physique selon la présente invention est obtenu par la voie aqueuse ou hydroalcoolique, avantageusement par la voie aqueuse telle que par exemple le procédé comprenant les étapes successives suivantes :
- préparation d'une solution de chitosane de concentration comprise entre 0,25% et 5% en poids par rapport au poids total de la solution, par mélange de chitosane purifié et lyophilisé et d'une solution aqueuse d'acide acétique ;
- gélification par contact de la solution obtenue avec des vapeurs d'ammoniac ; et
- lavage de l'hydrogel obtenu pour éliminer l'ammoniaque et les sels formés lors de la gélification. L'avantage de la voie aqueuse est que les hydrogels obtenus sont plus rapidement biodégradés dans le corps humain.

Les microparticules d'hydrogel de chitosan utilisables dans le cadre de la présente invention sont caractérisées par une taille médiane d50 comprise entre 1 et 500 µm (pour une distribution en nombre), avantageusement entre 5 et 300 µm, de façon avantageuse entre 10 et 50 µm, en particulier d'environ 20 µm. La taille médiane d50 des microparticles est mesurée par exemple par observation effectuée à l'aide d'un microscope optique à contraste de phase de marque Zeiss (Axiovert 200M), avec le logiciel d'acquisition d'image Linkys32. La distribution des tailles des microparticules d'hydrogel peut être obtenue par traitement d'image avec le logiciel ImageJ http://rsbweb.nih.gov/ij/, ou comme décrit dans C. Igathinathane et al (computers and electronics in agriculture 63 (2008) 168-182).

Les microparticules de chitosane ayant la taille médiane d50 désirée peuvent être obtenues par broyage de l'hydrogel, en particulier en utilisant un homogénéiseur ULTRATURAX de marque IKA (opérant à une vitesse de rotation de 11000 t/min pendant 3x 10s et un arrêt de 30 secondes entre les séquences) et récupération des microparticules de taille satisfaisante par exemple par centrifugation (par exemple 13000t/min pendant 3 minutes avec un appareil de type Sigma 3K30 de marque Bioblock Scientific).
Avantageusement, le procédé selon la présente invention comporte une étape de stérilisation, en particulier par autoclave, par exemple à 121°C pendant 20 minutes, avant la récupération des microparticules.

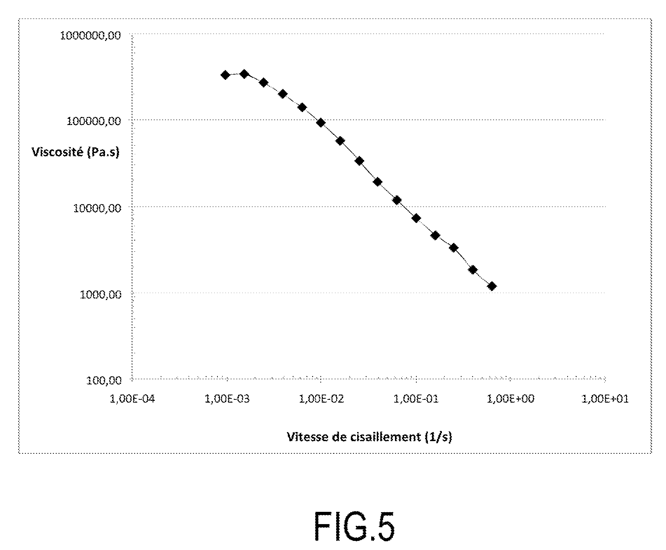

Dans un mode de réalisation particulier de la présente invention, les microparticules selon l'invention se trouvent sous la forme d'une suspension aqueuse, avantageusement ayant une viscosité supérieure à 1000 Pa.s, de façon avantageuse supérieure à 10 000 Pa.s, de façon encore plus avantageuse supérieure à 100 000 Pa.s, en particulier d'environ 330 kPa.s, (mesure en mode continu à 22°C) pour une vitesse de cisaillement de 0,001 s⁻¹, avantageusement mesurée par rhéométrie cone-plan avec un rhéomètre à contrainte imposée Advanced Rheometer AR2000 de marque TA instruments. C'est donc cette suspension qui sera implantée. Dans ce cas, l'implant utilisé comprend une suspension aqueuse contenant les microparticules d'hydrogel selon la présente invention. Avantageusement, la suspension est injectable ou implantable par chirurgie.
Par exemple, la valeur caractéristique de viscosité de la suspension peut être de 330 kPa.s après une centrifugation de 13000 t/min pendant 3 minutes à partir d'un hydrogel concentré à 2,5% en chitosane de masse molaire 450 000 g/mol (**Figure 5**).
Cette suspension doit donc être suffisamment liquide pour pouvoir être injectée directement dans le site lésionnel où les axones ont été coupés par l'impact qui a endommagé le tissu nerveux et suffisamment épaisse pour rester cantonnée dans le lieu d'implantation. L'avantage également que cette suspension soit injectable est de permettre une réparation dans le cas d'une contusion qui n'engendre pas nécessairement la rupture de la dure mère (la membrane qui protège les tissus nerveux juste en dessous de l'os (des vertèbres ou crâne)). Dans le cas du traumatisme crânien, l'impact peut endommager une zone en profondeur et une infection permet d'éviter d'induire une lésion importante supplémentaire. Cela peut également s'appliquer dans une lésion neurodégénérative (c'est-à-dire qui ne serait pas forcément due à un impact traumatique mais résultant d'une mort neuronale).
Ainsi cette suspension a plutôt la forme d'une pâte.

Dans un autre mode de réalisation de la présente invention, les microparticules selon l'invention sont mélangées avec des cellules de Schwann et/ou des cellules souches et/ou des facteurs trophiques.

Cette suspension peut être fabriquée par évaporation de l'eau à partir du culot de centrifugation contenant les microparticles selon la présente invention obtenu par le procédé de fabrication des microparticules indiqué ci-dessus, avantageusement de façon à obtenir une viscosité supérieure à 1000 Pa.s (mesure de la viscosité de la suspension en mode continu à 22°C pour une vitesse de cisaillement de 0,001 s⁻¹).
Cette évaporation peut, par exemple, se faire en déposant la suspension présente dans le culot de centrifugation sur une plaque de verre et en la laissant sécher quelques minutes (par exemple 5 à 10 min) à température ambiante (autour de 20°C) et à l'air libre jusqu'à obtenir la consistance requise pour la saisir avec une petite pince et la déposer dans le site de la lésion lors d'une chirurgie. Cela est valable dans le cas d'une lésion traumatique de la moelle épinière. Dans le cas d'une injection, le culot peut être resuspendu dans du liquide physiologique ou liquide céphalorachidien artificiel.

La présente invention concerne, en outre, un implant comprenant une suspension aqueuse de microparticules telle que définie ci-dessus et des cellules de Schwann et/ou des cellules souches et/ou des facteurs trophiques. L'addition des cellules peut se faire une à deux heures avant l'implantation, le temps qu'elles adhèrent aux microparticules étant donné que le chitosane a des propriétés adhésives que les inventeurs avaient d'ailleurs vérifié in vitro, sur des cultures de cellules. De façon avantageuse, cet implant ne contient pas de chondrocytes.
De façon avantageuse, cet implant est un implant destiné au système nerveux, en particulier au système nerveux central, avantageusement à la moelle épinière. Il ne doit donc pas susciter de réaction inflammatoire dans le lieu d'implantation, supplémentaire à celle crée par l'impact lésionnel.

L'avantage des microparticles selon la présente invention est qu'elles fournissent une surface appropriée pour l'adhésion aussi bien cellulaire et axonale d'une part et d'autre part que les axones peuvent utiliser la porosité inter-particulaire (entre les microparticules de la suspension) pour envahir le site lésionnel En effet, les inventeurs ont déjà vérifié que des neurones mis en culture poussent leurs neurites (entre autres axones) sur un substrat composé de la même suspension de chitosane proposée ici pour l'implantation in vivo. Pour pousser, les axones ont besoin d'une attache pour créer une force d'extension.

La suspension ou les microparticules peuvent également être utilisées dans des systèmes plurimembranaires tels que par exemple les fibres plurimembranaires de chitosane décrites dans la demande de brevet WO2009/044053 pour la constitution d'implants, par exemple au niveau de la lésion traumatique.

La présente invention concerne en outre une méthode de régénération neuronale et/ou de réparation du système nerveux (central ou périphérique), avantageusement du système nerveux central (en particulier la moelle épinière), et/ou de greffe de neurones ou de précurseurs de cellules neurales (glie et neurones) et/ou de traitement des maladies neurodégénératives (telles que la maladie d'Afzheimer ou de Parkinson, ou encore la sclérose en plaques) et/ou lésion ischémique et/ou de traitement des paralysies comprenant l'implantation de microparticles selon la présente invention chez un patient nécessitant un tel traitement, en particulier au niveau d'une lésion avantageusement traumatique afin de combler cette lésions, ou à l'endroit où la repousse axonale ou la réparation ou la greffe doit avoir lieu. Avantageusement, cette implantation se fait par dépôt, injection ou chirurgie.

La présente invention sera mieux comprise à la lumière des figures et des exemples qui survent.

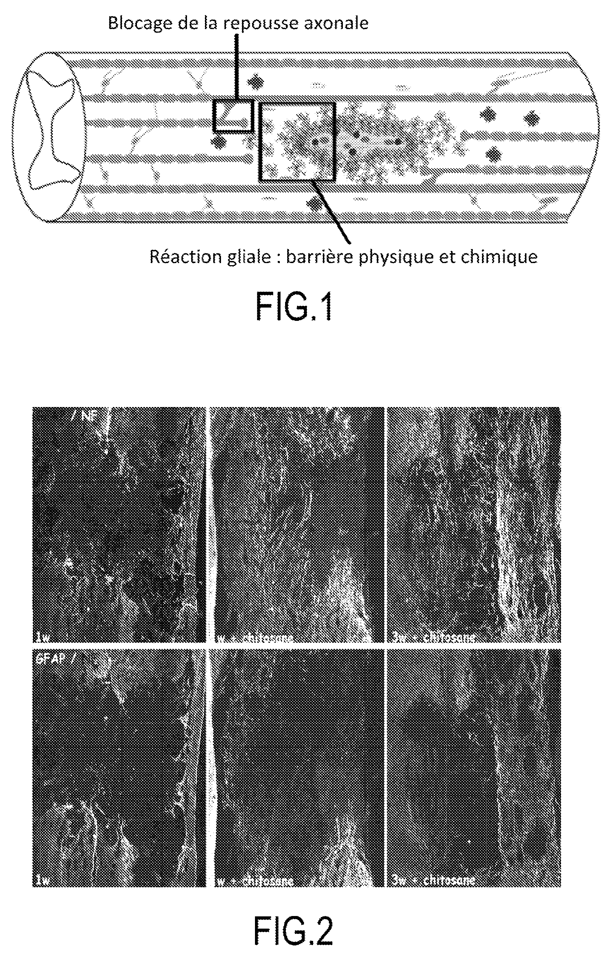

La **Figure 1** représente le schéma d'une lésion traumatique dans la moelle épinière. Ce schéma montre qu'autour du site lésionnel initial, les axones qui ont été coupés, sont bloqués et ne repoussent pas (beaucoup d'entre eux se rétractent ou dégénèrent au cours du temps). Les astrocytes migrent et entourent la lésion pour former la barrière physique et chimique, sans pour autant migrer à l'intérieur du site lésionnel. La cicatrisation est souvent suivie par la formation d'une cavité (observée chez l'homme et le rat par exemple).
La **Figure 2** représente des photographies de doubles immunofluorescences réalisées sur des coupes de moelle épinière de rat adulte, 1 semaine (1w, figures à gauche et au milieu) et 3 semaines (figures à droite, 3w) après hémisection, avec implantation de microparticles selon l'exemple 1 de la présente invention (+chitosane, figures au milieu et à droite) ou sans implantation (la plus à gauche). Les photographies du haut montrent le marquage des neurofilaments (NF) qui met en évidence la présence des axones des neurones. Les photographies du bas (GFAP) montrent les astrocytes (cellules gliales du système nerveux central). A noter qu'à une semaine, de nombreux axones arrivent du tissu hôte et ont déjà envahi le site lésionnel, aussi bien du côté rostral (vers le cerveau) que du côté caudal (vers la queue de l'animal). Comparer la densité des axones à une semaine entre la coupe avec lésion seule et la coupe avec une lésion+chitosane. En regardant les figures de 1 et 3 semaines lésion+chitosane, on peut remarquer que le nombre d'axones a encore augmenté au cours du temps et la repousse s'est faite sur de longues distances. A noter également que la réaction gliale reste faible.

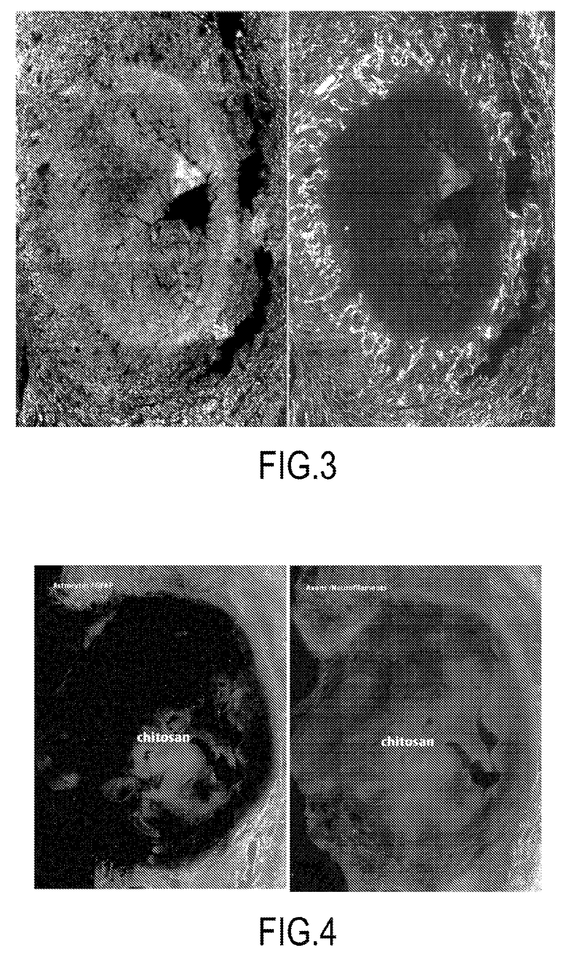

La **Figure 3** représente une photographie d'une coupe de moelle épinière de rat adulte, 4 semaines après hémisection et implantation de microparticules d'hydrogel physique de chitosane ayant un degré d'acétylation élevé (35%) au niveau du site de la lésion traumatique. La photographie de gauche comporte un double marquage avec l'anti-ED1 des macrophages et la photographie de droite un immunomarquage de la laminine qui met en évidence les micro-vaisseaux sanguins et donc la vascularisation. Ce type d'implant crée une forte réaction inflammatoire, bloque la repousse axonale et même la migration des cellules endothéliales (cellules à l'origine des vaisseaux sanguins) à travers l'implant, alors qu'à bas degré d'acétylation une vascularisation est notée à l'intérieur de l'implant de microparticules.
La **Figure 4** représente une photographie d'une coupe de moelle épinière de rat adulte, 4 semaines après hémisection et implantation d'un bloc d'hydrogel physique de chitosane à bas degré d'acétylation (3 %) au niveau du site de la lésion traumatique. La photographie de gauche comporte un immunomarquage avec un anti-GFAP des astrocytes et la photographie de droite un immunomarquage des axones par un anti-Neurofilament. Même si la réaction gliale astrocytaire et la réaction inflammatoire sont réduites, les axones à proximité de l'implant, se trouvent bloqués à la frontière et ne repoussent pas à travers l'implant.
La **Figure 5** représente la courbe d'évolution de la viscosité (en Pa.s) d'une suspension de microparticules selon l'exemple 1 de l'invention (obtenue après centrifugation à 1300 tours/ minutes pendant 3 minutes, l'hydrogel comprenant 2,5% en poids de chitosane ayant un degré d'acétylation de 3%) en fonction de la vitesse de cisaillement (en s⁻¹), la viscosité étant mesurée en mode continu à 22°C par rhéométrie cone-plan avec un rhéomètre à contrainte imposée Advanced Rheometer AR2000 de marque TA instruments.

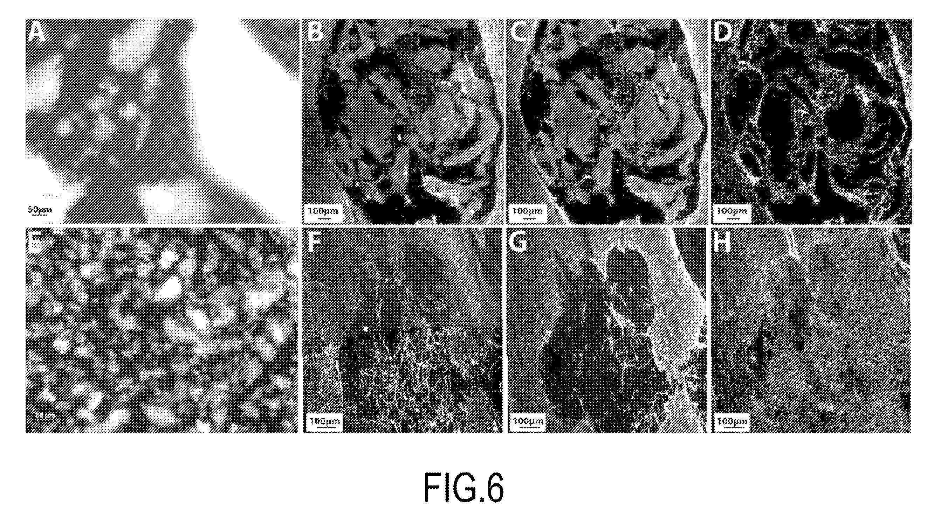

La **Figure 6** permet d'observer, en microscopie optique à contraste de phase, sur la première ligne une dispersion de microparticules d'hydrogel physique de chitosane selon l'exemple comparatif 3 et sur la deuxième ligne selon un exemple réalisé conformément à l'exemple 1.

**A-** Cette photographie permet de mesurer la distribution de taille des microparticules de gel avant implantation, avec une grande dimension excédant 500 microns pour une fraction majoritaire de particules, correspondant donc à une d50 supérieure à 500 µm. Quatre semaines après implantation de cette formulation dans la moelle épinière de rat adulte ayant subi une hémisection (**B**-**D**), on peut noter que le polymère reste très opaque en microscopie optique à contraste de phase ; très peu d'axones (marquage spécifique des axones en B), très peu d'astrocytes (marquage spécifique des astrocytes en C) envahissent l'implant. De plus, le marquage des noyaux des cellules (marquage spécifique des noyaux cellulaires en D) montre que peu de cellules envahissent l'implant, avec des zones occupées par les microgels non colonisées.

**E-** Cette photographie permet de mesurer la distribution de taille des microparticules de gel avant implantation, avec une taille médiane apparente de fragments comprise entre 20 et 50 microns. Quatre semaines après implantation de ce cette formulation dans la moelle épinière de rat adulte ayant subit une hémisection (**F**-**H**), on peut noter que la lésion est envahie par de nombreuses cellules (marquage spécifique des noyaux cellulaires en **H),** parmi lesquelles des astrocytes (marquage spécifique des astrocytes ou cellules en étoiles en **G)** la réaction astrocytaire qui entoure l'implant est très peu marquée (**G**), à l'inverse de ce qui peut être observé avec les microparticules de chitosane sur la Figure **6 -C**. De plus, de nombreux axones (marquage spécifique des axones en **F)** envahissent l'implant.

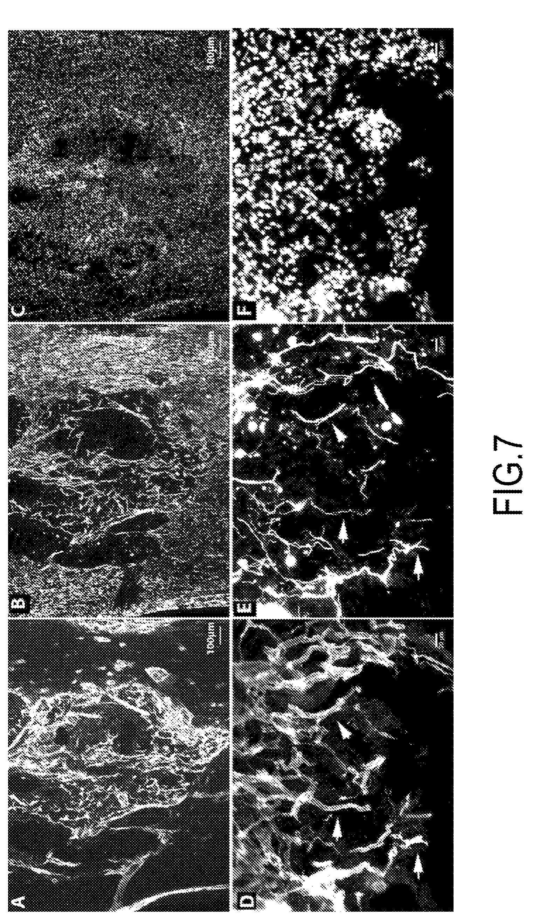

La **Figure 7** présente des photographies d'une coupe de moelle épinière de rat adulte montrant l'évolution de la lésion après implantation de microparticules de chitosane selon l'invention de la **Figure 6 - E****, 4** semaines après la lésion et l'implantation. **A**-**C** ; triple marquages montrant une néo-vascularisation (marquage spécifique des vaisseaux en **A)** très organisée dans l'implant, accompagnée d'une forte repousse des axones (marquage spécifique des axones en **B)** et d'une invasion massive par des cellules (marquage spécifique des noyaux cellulaires **C). D**-**E** : fort grossissement réalisés dans l'implant qui montrent l'association (flèches) des axones **(D)** avec les microvaisseaux **(E)** nouvellement formés dans l'implant.

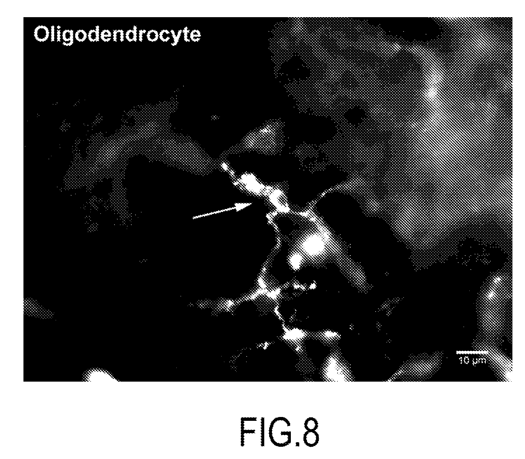

La **Figure 8** présente une photographie d'une coupe de moelle épinière de rat adulte montrant l'évolution de l'implant après implantation de microparticules de chitosane selon l'invention de la **Figure 6 - E.** Parmi les cellules qui colonisent l'implant, on note des oligodendrocytes (flèche), cellules qui pourront myéliniser les axones en régénération, et ainsi rétablir l'influx nerveux.

### Exemple 1 : fabrication de microparticules selon la présente invention

Dissolution du chitosane de degré d'acétylation de 3 % provenant de chitine de calamar commercialisé par la société Mahtani Chitosan, dans une solution aqueuse d'acide acétique (introduit en quantités stoechiométriques par rapport aux fonctions aminé) de façon à obtenir une solution contenant 0,5 % en poids de chitosane.
Filtration sur filtres à 3 microns, 1 microns et 0,45 microns.
Précipitation par la soude ou ammoniac jusqu'à atteindre un pH de 14. Récupération du précipité par centrifugation.
Lavage à l'eau déionisée jusqu'à ce que le pH des eaux de lavage soit neutre pour l'élimination des sels.
Lyophilisation du précipité lavé pour obtenir un produit sec.
Préparation d'une solution de chitosane de masse molaire de 450 000 g/mol à 2,5% en poids par rapport au poids total de la solution à partir du précipité et d'une eau pure (Versol®).
Gélification par contact de la solution dans des boites de Pétri de diamètres de quelques centimètres, en présence de vapeurs d'ammoniac (72h).
Lavage des hydrogels à l'eau déionisée pour l'élimination d'ammoniac. Renouvellement de l'opération pour un total de 7 lavages.
Vérification de l'atteinte du pH de neutralité.

Broyage des hydrogels avec l'appareil ULTRATURAX de marque IKA (opérant à une vitesse de rotation de 11000 t/min pendant 3x 10s et un arrêt de 30 secondes entre les séquences).
Stérilisation des microparticules d'hydrogel par autoclave (121°C pendant 20 minutes).
Centrifugation (13000 t/min pendant 3 minutes avec un appareil de type Sigma 3K30 de marque Bioblock Scientific) de façon à récupérer les microparticules stérilisées ayant une taille médiane d50 d'environs 20 µm. Ces microparticules servent ensuite pour la fabrication d'une suspension aqueuse de viscosité minimale de 1000Pa.s. Pour cela, on dépose le culot de centrifugation obtenu sur une plaque de verre que l'on laisse sécher à l'air libre à température ambiante pendant quelques minutes. Il se produit un séchage partiel qui permet d'augmenter la viscosité jusqu'à une viscosité de 330 kPa.s mesurée à 22°C pour une vitesse de cisaillement de 0,001 s⁻¹ (mesure en mode continu par rhéométrie cone-plan avec un rhéomètre à contrainte imposée Advanced Rheometer AR2000 de marque TA instruments) comme indiqué dans la **Figure 5****.**

### Exemple comparatif 1 : fabrication de microparticles d'hydrogel physique de chitosane de fort degré d'acétylation

On utilise le même procédé que celui décrit dans l'exemple 1 (les microparticules obtenues ont la même taille médiane d50 que dans l'exemple 1, la teneur en chitosan est la même que dans l'Exemple 1, de même que la viscosité de la suspension obtenue) à part que le chitosane de départ 3 un degré d'acétylation de 35%. Pour obtenir un tel degré d'acétylation, le chitosane a été réacétylé à partir d'un chitosane de bas degré d'acétylation (3%) acheté chez Mahtani Chitosan et séché comme indiqué dans l'exemple 1, en milieu hydroalcoolique, comme décrit dans la publication suivant : Biomacromolecules. 2001 2(3):765-72*. Relation between the degree of acetylation and electrostatic properties of chitin and chitosan. Sorlier P, Denuzière A, Viton **C***, *Domard A.*

### Exemple comparatif 2 : fabrication de blocs d'hydrogel physique de chitosane de faible degré d'acetylation

On utilise le même procédé que celui décrit dans l'exemple 1 à part qu'il n'y a pas d'étape de broyage, de centrifugation et de séchage partiel sur lame de verre. L'hydrogel physique obtenu est directement stérilisé avant d'être découpé dans les dimensions voulues.

### Exemple comparatif 3 : fabrication de microparticules présentant une d50 supérieure à 500 µm

Le procédé d'obtention de ces particules est identique à celui de l'exemple 1, à part que la concentration en chitosane dans l'hydrogel de départ est de 3,5% en poids de chitosane par rapport au poids total, et que la durée de broyage du gel est de seulement 10 secondes, au lieu de 3x 10 secondes.

### Essai de réparation de lésion traumatique dans la moelle épinière

Pour ces essais, la lésion de la moelle épinière est réalisée par une hémisection latérale en thoracique Th8/Th9 de la ME d'un rat adulte suivie par un prélèvement de la partie dorsale latérale (2 à 3 mm) du segment découvert. Ce type de lésion se trouve entre le dernier segment thoracique et le premier segment lombaire. Dans le cas d'une hémisection, cela engendre une hémiplégie de la patte postérieure du même côté de la lésion. Des rats témoins subissent le même type de lésion mais sont laissés à cicatriser sans implant.
Les implants ont été introduits par chirurgie immédiatement après la réalisation de la lésion et réduction de saignement provoqué par le traumatisme. Les implants mis à l'essai sont de trois types :
- une quantité représentant environ 2-3 mm³ de la suspension aqueuse de microparticules selon la présente invention (obtenue selon l'exemple 1)
- une quantité représentant environ 2-3 mm³ de la suspension aqueuse de microparticules d'hydrogel de chitosane de fort degré d'acétylation

### (obtenue selon l'exemple comparatif 1)

- ou un bloc d'environ 2-3 mm³ découpé dans l'hydrogel physique de chitosane obtenu selon l'exemple comparatif 2.

Au total 24 animaux (12 avec lésion + implant et 12 contrôles: lésion uniquement) ont été analysés.

A des temps variables post-lésionnels, les animaux sont profondément anesthésiés et perfusés à partir du coeur avec un fixateur (4% de paraformaldéhyde dans du tampon phosphate 0,1 M, PBS) pour fixer le tissu d'intérêt. Des coupes de moelle épinière sont alors effectuées entre 1 (n=8) et 3 (n=8) à 4 (n=8) semaines post-lésionnelles, de façon à pratiquer des analyses morphologiques et immunohistologiques.

Après dissection et prélèvement, les tissus d'intérêt (moelle épinière) sont cryoprotégés avec du sucrose pour réaliser des coupes de 30 mm au cryostat qui sont montées sur lames et conservées à -80°C jusqu'à leur utilisation en histologie.

Après perméabilisation (Triton 0,3% dans PBS) et saturation des sites aspécifiques (NGS (normal Goat Serum), 10% dans PBS), les coupes sont incubées dans la solution d'anticorps primaires diluée dans NGS 5%-PBS, toute la nuit à 4°C. L'incubation avec les anticorps secondaires couplés aux fluorochromes appropriés est réalisée pendant 2h à température ambiante, à l'abri de la lumière. Après rinçage, les lames sont montées au Mowiol. Les marquages sont analysés par microscopie à fluorescence. Comme illustré dans la **Figure 1** une lésion mécanique dans la moelle épinière entraîne la formation d'une barrière physique et chimique qui entoure la lésion et bloque la repousse des axones lésés.

La **Figure 2** montre, dans les trois photographies du bas, des astrocytes (cellules gliales du système nerveux central) qui s'activent suite à la lésion et qui sont responsables de la formation de la barrière illustrée dans la **Figure 1****.** La **Figure 2** montre, dans la première photographie du haut, que les axones des neurones ne pénètrent pas la lésion délimitée par la barrière, à 1 semaine sans implant de chitosane,

L'implantation de microparticules de chitosane selon la présente invention permet en revanche une croissance massive des axones au travers de la lésion, à 1 et 3 semaines (noter le fort marquage fibrillaire au centre de la lésion et l'orientation rectiligne de la repousse axonale à travers le site de la lésion) comme le montre les deux dernières photos en haut, à droite (NF) de la **Figure 2****.**

Les analyses morphologiques et immunohistologiques montrent donc clairement, et d'une façon impressionnante, les changements qui ont lieu après implantation des microparticules selon l'invention dans la ME lésée, en comparaison à une lésion seule sans implant:
- La réaction gliale astrocytaire est nettement réduite. Le corps cellulaire des astrocytes est moins atrophié, leurs processus sont plus fins et plus longs, très souvent orientés vers le centre de la lésion. Cela rend la frontière tissu intact-lésion moins nette, par manque d'accumulation des processus astrocytaires entourant le site lésionnel, donc réduction de la barrière physique. Cela démontre qu'il existe une grande compatibilité entre le tissu hôte et l'implant. Par ailleurs, cet aspect morphologique des astrocytes -orientation de leurs prolongements vers l'épicentre de la lésion- est un signe que ces cellules jouent plutôt un rôle favorable pour la repousse des axones. D'ailleurs, à l'entrée de l'implant, on observe souvent les prolongements des astrocytes associées avec les axones en repousse avec la même orientation (parallèle) et ce sur une longue distance. Une observation importante qui renforce cette compatibilité est la présence des astrocytes (identification de leur corps cellulaire) au sein de l'implant, preuve que ces cellules ont également migré à l'intérieur de l'implant. Il apparaît donc que les microparticules de chitosane selon l'invention forment un substrat permissible pour ce type de cellules gliales.
- Malgré la grande taille de la lésion (3 à 4 mm de large), l'introduction des microparticules n'augmente pas la réaction inflammatoire (macrophagique) et la cavité cystique est réduite.
- Par le marquage nucléaire au **DAPI**, il a pu être noté que les microparticules d'hydrogel de chitosane sont habitées par de nombreuses cellules : les macrophages/microglie ; les astrocytes comme indiqué ci-dessus ; les cellules endothéliales qui forment de nouveau vaisseaux. A ce propos, l'implant est bien vascularisé et le réseau ou la cytoarchitecture de la néovascularisation se fait d'une façon plus organisée (comme le témoigne la **Figure 6****).** Dans l'implant et dans le tissu hôte environnant le site de la lésion, de nombreux précurseurs des oligodendrocytes (cellules qui myélinisent les axones du système nerveux central) ont également été identifiées, ce qui montre qu'en présence de l'implant la prolifération des NPCs (multipotent neural precursor cells) endogènes est stimulée et leur différentiation vers le phénotype des oligodendrocytes est favorisée. Cette observation est importante dans la mesure où on sait que suite à une lésion, une démyélinisation a lieu même au niveau des axones qui ne sont pas directement endommagés. Cela suggère que, par la stimulation de la prolifération et la différenciation des oligodendrocytes la réparation de remyélinisation peut avoir lieu et, par conséquence, l'activité électrique neuronale rétablie (comme le témoigne la **Figure 8**). De telles observations présagent donc de l'intérêt des microparticules d'hydrogel de chitosane selon l'invention pour le traitement de la sclérose en plaque.
- Le plus remarquable est la présence d'un nombre important d'axones qui traversent le site lésionnel, comme le témoigne la **Figure 2****.** Ces axones pénètrent dans l'implant aussi bien en amont qu'en aval de la lésion. La **Figure 2** témoigne également, que la capacité de repousse se fait sur une longue distance. Ces observations sont notées à 1 et 4 semaines post-lésionnelles, et ont été confirmées au-delà de 3 mois post-lésionnels. Il est également intéressant de noter que la repousse axonale dans l'implant est associée avec la vascularisation (comme le témoigne la **Figure 7**). En effet, il a été montré, dans un modèle de lésion de très petite taille et par imagerie biphotonique in vivo sur l'animal sous anesthésie, que les axones qui tentent de régénérer sont guidés (où aidés) au début de leur repousse par les vaisseaux environnant (Dray C, Rougon G, Debarbieux F, Proc Natl Acad Sci U S A. 2009 Jun 9;106(23):9459-64. dol: 10.1073/pnas.0900222106. Epub 2009 May 21). Dans le cas présent, on observe cette association (sur tissu fixé) après plusieurs semaines. Il est à noter que l'approche in vivo sur l'animal après une lésion sévère de la moelle épinière n'est pas faisable.

L'ensemble de ces observations au niveau de l'implant témoigne que les microparticules de chitosane selon l'invention constituent un substrat, permissif et attractif, très favorable pour restorer la moelle épinière traumatique. Son implantation locale dans le site de la lésion crée un environnement favorable pour l'ensemble des cellules neurales et s'avère un substrat permissif et attractif pour la pousse axonale. Par ailleurs, l'invasion de l'implant par les cellules endogènes et l'établissement d'une vascularisation permettent de recréer un pont tissulaire entre la partie rostrale (vers la tête) et caudale et d'empêcher qu'une nécrose se forme qui, dans le cas de la lésion seule, aboutit à la formation d'une cavité entourée par une frontière asctrocytaire et moléculaire pour protéger le tissus sein d'une propagation du dommage. Un tel effet n'a jamais été montré, même dans les stratégies combinant d'autres approches.

Au contraire, la **Figure 3** montre, sur la photographie de gauche, une réaction inflammatoire (avec l'anti-ED1) (noter la couronne de l'immunomarquage autour du chitosane) et sur la photographie de droite que la vascularisation (par immunomarquage de la laminine) se limite à l'extérieur du chitosane, Ainsi, ceci prouve que la repousse axonale est bloquée et que les réactions astrocytaire et inflammatoire sont importantes. L'importante réaction inflammatoire a également engendré une très forte néoangiogenèse et une lésion secondaire plus grande.

Enfin, la **Figure 4** montre que la microstructure de l'implant a un impact très sensible sur la réponse tissulaire. En effet Il est nécessaire d'utiliser des microparticules et non un hydrogel monolithique, massif ou en bloc : dans ce dernier cas la repousse axonale est bloquée à la frontière du bloc de chitosane par la présence du matériau.

Ainsi donc, ces essais montrent de façon tout à fait surprenante qu'une suspension de microparticules d'hydrogel physique de chitosane de faible degré d'acétylation permet de stimuler et guider la repousse axonale dans les lésions traumatiques de la moelle épinière, sans susciter d'inflammation.

## Revendications

1. Microparticules d'hydrogel de chitosane de taille médiane d50, obtenue à partir d'une distribution en nombre, comprise entre 1 et 500 µm, le chitosane ayant un degré d'acétylation inférieur ou égal à 20 % et sa concentration dans l'hydrogel étant comprise entre 0,25 et 5% en poids par rapport au poids total de l'hydrogel, pour leur utilisation dans la régénération neuronale et/ou dans la réparation du système nerveux, avantageusement du système nerveux central, et/ou dans la greffe de neurones ou de précursseurs de cellules neurales et/ou dans le traitement des maladies neurodégénératives et/ou dans le traitement des lésions ischémiques et/ou dans le traitement des paralysies.

2. Microparticules selon la revendication 1 **caractérisées en ce que** la réparation, la régénération ou le traitement des paralysies fait suite à une lésion traumatique du système nerveux, avantageusement de la moelle épinière.

3. Microparticules selon l'une quelconque des revendications 1 ou 2, **caractérisées en ce que** leur taille médiane d50, obtenue à partir d'une distribution en nombre, est comprise entre 5 et 300 µm.

4. Microparticules selon l'une quelconque des revendications 1 à 3, **caractérisées en ce que** le degré d'acétylation du chitosane est inférieur à 5%.

5. Microparticules selon l'une quelconque des revendications 1 à 4, **caractérisées en ce que** la concentration du chitosane dans l'hydrogel est inférieure à 4 % en poids par rapport au poids total de l'hydrogel.

6. Microparticules selon l'une quelconque des revendications 1 à 5, **caractérisées en ce que** l'hydrogel est un hydrogel physique de chitosane.

7. Microparticules selon l'une quelconque des revendications 1 à 6, **caractérisées en ce qu'**elles se trouvent sous la forme d'une suspension aqueuse, avantageusement ayant une viscosité supérieure à 1000 Pa.s mesurée en mode continu à 22°C pour une vitesse de cisaillement de 0,001 s⁻¹,

8. Microparticules selon la revendication 7 **caractérisées en ce que** la suspension est injectable ou implantable par chirurgie.

9. Microparticules selon l'une quelconque des revendications 7 ou 8, **caractérisées en ce qu'**elles sont mélangées avec des cellules de Schwann et/ou des cellules souches et/ou des facteurs trophiques.

10. Microparticules selon l'une quelconque des revendications 1 à 9 pour le traitement de la maladie d'Alzheimer, de la maladie de Parkinson, ou de la sclérose en plaque.

11. Implant comprenant une suspension aqueuse de microparticules telles que définies dans la revendication 9.

## Patentansprüche

1. Mikropartikel aus Chitosan-Hydrogel mit einer mittleren Größe d50, erhalten anhand einer Zahlenverteilung, zwischen 1 und 500 µm, wobei das Chitosan einen Acetylierungsgrad von weniger als oder gleich 20 % aufweist und seine Konzentration in dem Hydrogel zwischen 0,25 und 5 Gew.-% bezogen auf das Gesamtgewicht des Hydrogels beträgt, für deren Verwendung bei der neuronalen Regeneration und/oder bei der Reparatur des Nervensystems, vorteilhafterweise das Zentralnervensystems, und/oder bei der Transplantation von Neuronen oder von Vorläufern von Nervenzellen und/oder bei der Behandlung von neurodegenerativen Erkrankungen und/oder bei der Behandlung von ischämischen Schädigungen und/oder bei der Behandlung von Lähmungen.

2. Mikropartikel nach Anspruch 1, **dadurch gekennzeichnet, dass** die Reparatur, die Regeneration oder die Behandlung von Lähmungen auf eine traumatische Verletzung des Nervensystems, vorteilhafterweise des Rückenmarks folgt.

3. Mikropartikel nach einem der Ansprüche 1 oder 2, **dadurch gekennzeichnet, dass** ihre mittlere Größe d50, erhalten anhand einer Zahlenverteilung, zwischen 5 und 300 µm beträgt.

4. Mikropartikel nach einem der Ansprüche 1 bis 3, **dadurch gekennzeichnet, dass** der Acetylierungsgrad des Chitosan weniger als 5 % beträgt.

5. Mikropartikel nach einem der Ansprüche 1 bis 4, **dadurch gekennzeichnet, dass** die Konzentration des Chitosan in dem Hydrogel weniger als 4 Gew.-% bezogen auf das Gesamtgewicht des Hydrogels beträgt.

6. Mikropartikel nach einem der Ansprüche 1 bis 5, **dadurch gekennzeichnet, dass** das Hydrogel ein physikalisches Chitosan-Hydrogel ist.

7. Mikropartikel nach einem der Ansprüche 1 bis 6, **dadurch gekennzeichnet, dass** sie sich in Form einer wässrigen Suspension, vorteilhafterweise mit einer Viskosität von über 1000 Pa.s, gemessen im kontinuierlichen Modus bei 22 °C bei einer Schergeschwindigkeit von 0,001 s⁻¹, befinden.

8. Mikropartikel nach Anspruch 7, **dadurch gekennzeichnet, dass** die Suspension injizierbar oder chirurgisch implantierbar ist.

9. Mikropartikel nach einem der Ansprüche 7 oder 8, **dadurch gekennzeichnet, dass** sie mit Schwann-Zellen und/oder Stammzellen und/oder Nahrungsfaktoren gemischt werden.

10. Mikropartikel nach einem der Ansprüche 1 bis 9, für die Behandlung von Alzheimer-Krankheit, von Parkinson-Krankheit oder von Multiple Sklerose.

11. Implantat, das eine wässrige Suspension von Mikropartikeln, wie sie in Anspruch 9 definiert sind, umfasst.

## Claims

1. Microparticles of chitosan hydrogel of a median size d50, obtained from a number distribution, comprised between 1 and 500 µm, the chitosan having a degree of acetylation of less than or equal to 20% and its concentration in the hydrogel being comprised between 0.25 and 5% by weight based on the total weight of the hydrogel, for their use in neuron regeneration and/or in the repair of the nervous system, advantageously of the central nervous system, and/or in the grafting of neurons or precursors of neural cells and/or in the treatment of neurodegenerative diseases and/or in the treatment of ischemic wounds and/or in the treatment of paralyses.

2. The microparticles according to claim 1, **characterized in that** the repair, regeneration or treatment of paralyses is subsequent to a traumatic lesion of the nervous system, notably of the spinal cord.

3. The microparticles according to any of claims 1 or 2, **characterized in that** their median size d50, obtained from a number distribution, is comprised between 5 and 300 µm.

4. The microparticles according to any of claims 1 to 3, **characterized in that** the degree of acetylation of the chitosan is less than 5%.

5. The microparticles according to any of claims 1 to 4, **characterized in that** the concentration of the chitosan in the hydrogel is less than 4% by weight based on the total weight of the hydrogel.

6. The microparticles according of any of claims 1 to 5, **characterized in that** the hydrogel is a physical chitosan hydrogel.

7. The microparticles according to any of claims 1 to 6, **characterized in that** they are found as an aqueous suspension, advantageously having the viscosity greater than 1000 Pa.s measured in a continuous mode at 22°C for a shear rate of 0.001 s⁻¹.

8. The microparticles according to claim 7, **characterized in that** the suspension is injectable or implantable by surgery.

9. The microparticles according to any of claims 7 or 8, **characterized in that** they are mixed with Schwann cells and/or stem cells and/or trophic factors.

10. The microparticles according to any of claims 1 to 9, for treating Alzheimer's disease, Parkinson's disease, or multiple sclerosis.

11. An implant comprising an aqueous suspension of microparticles as defined in claim 9.
